# FASCICULE DE BREVET EUROPEEN

(11) **EP 2 385 812 B1**
(45) Date de publication et mention de la délivrance du brevet: **23.11.2016**
(21) Numéro de dépôt: 10702516.5
(22) Date de dépôt: 08.01.2010
(51) Int. Cl.: A61F 2/42

(54) **TIGE D'ANCRAGE INTRA MÉDULLAIRE POUR TÊTE D'IMPLANT ORTHOPÉDIQUE**
INTRAMEDULLÄRER VERANKERUNGSSCHAFT FÜR EINEN ORTHOPÄDISCHEN IMPLANTATKOPF
INTRAMEDULLARY ANCHORING STEM FOR AN ORTHOPAEDIC IMPLANT HEAD

(30) Priorité: 08.01.2009 FR 0900053
(43) Date de publication de la demande: 16.11.2011
(73) Titulaire: Stryker European Holdings I, LLC, Kalamazoo, MI 49002 (US)
(72) Inventeur: BELLEMERE, Philippe, 44000 Nantes (FR); DREANO, Thierry, 35000 Rennes (FR); GOUBAU, Jean, 8210 Loppem (BE); MARTINACHE, Xavier, 51000 Reims (FR); PAPALOIZOS, Michaël, 1204 Genève (CH); PRANDI, Bernard, 35700 Rennes (FR); SIRET, Pierre, 35160 Talensac (FR); TCHURUKDICHIAN, Alain, 21000 Dijon (FR)
(74) Mandataire: Regimbeau
(86) Numéro de dépôt international: PCT/FR2010/000011
(87) Numéro de publication internationale: WO 2010/079289

(56) Documents cités:
- EP-A- 1 632 200
- FR-A- 2 736 818
- FR-A- 2 912 051
- US-A- 5 425 777
- US-A1- 2003 040 805

## Description

La présente invention concerne un implant orthopédique utilisé en arthroplastie digitale.

Elle trouve une application particulièrement importante bien que non exclusive dans le domaine médical des prothèses de l'articulation Inter-Phalangienne Proximale de la main (connue dans le domaine par ses initiales IPP).

Mais elle est également applicable plus généralement aux articulations entre d'autres types de phalange dite proximale et une phalange adjacente distale.

Par phalange dite proximale on entend une phalange ou un élément osseux métacarpien ou métatarsien situé du côté du corps ou du membre (main ou pied) concerné, et par phalange distale une phalange située vers l'extérieur de ce membre, selon les conventions d'orientation adoptée classiquement en anatomie, et qui seront par ailleurs utilisées ci-après.

L'invention peut ainsi être utilisée pour des prothèses articulaires digitales Inter-Phalangiennes du pouce (IP pouce) ou pour des prothèses Inter-Phalangiennes Distales (IPD), pour la main ou pour le pied.

Elle peut aussi être avantageusement utilisée pour des prothèses articulaires digitales méta-carpophalangiennes (dits MCP), trapezo-métacarpiennes (dits MTP), ici encore pour la main ou pour le pied, et ce par simple adaptation géométrique aux sites osseux concernés.

On connaît déjà des prothèses d'articulation inter-phalangiennes comprenant des premiers et des seconds éléments comportant chacun respectivement une tige d'implantation dans l'os et une tête d'articulation inter-phalangienne complémentaire.

Par exemple il est connu (EP 1 339 362) un implant comportant un premier élément muni d'une tête présentant une surface convexe d'articulation bi-condylienne et un second élément muni d'une tête complémentaire.

Les éléments de telles prothèses sont constitués d'une pièce, la tige présentant une forme de pieu plein solidaire de la tête et agencé pour être introduit à l'intérieur de la cavité médullaire d'une phalange.

Le problème résolu par ce type de prothèse est celui de l'usure des surfaces articulaires, la tige elle-même étant considérée comme secondaire.

On connaît également (EP 1 870 061), des prothèses comportant des tiges d'ancrage coniques allongées autour d'un axe.

Ici encore le problème de l'usure des prothèses est strictement résolu grâce à une adaptation des surfaces d'articulation entre elles.

On connaît aussi (US 2003/0040805) des prothèses en trois parties à savoir, une tête, une pièce d'écartement et une tige creuse dont l'extrémité est en forme de bec de canard formant deux lamelles flexibles déformables entre une position d'introduction dans l'os et une position d'ancrage, où la pièce d'écartement bloque la prothèse. Un tel système est compliqué à mettre en oeuvre.

La présente invention qui est définie dans la revendication 1 vise à fournir un implant orthopédique répondant mieux que ceux antérieurement connus aux exigences de la pratique notamment en ce qu'elle engendre une usure très faible malgré de nombreux mouvements d'articulation, et en ce qu'elle présente une grande simplicité et une grande facilité de montage en s'adaptant aux contraintes liées aux fonctionnements ultérieurs des articulations arthroplastiques.

Pour ce faire, elle part notamment d'une approche différente de celle de l'art antérieur.

Plutôt que de prendre pour acquis la nécessité d'une tige d'implantation pleine et solidement rigide, pour assurer l'ancrage d'une articulation dont on limite ensuite l'usure par l'adoption d'une forme particulière des têtes, la présente invention a tout d'abord accepté la remise en cause de la conception même de la tige solidaire de la tête.

Elle l'a donc en premier lieu rendue amovible par rapport à la tête, ce qui permet notamment l'utilisation de deux matériaux différents et le remplacement de la tête sans avoir à retirer la tige.

La tige peut être d'une seule pièce notamment propre à être implantée, sans vissage dans le canal intra-médullaire de l'os.

L'invention a enfin et en combinaison avec cette dissociation et liaison rigide d'un seul tenant, rendu la tige elle même plus flexible dans le plan dorso-palmaire en proposant une tige présentant une élasticité apparente et une rigidité proche de l'os dans lequel elle est implantée.

L'ensemble de ces éléments pris en combinaison va entraîner un comportement plus naturel des têtes contre elles, avec pour conséquence inattendue de limiter considérablement leur usure tout en présentant les avantages des monopièces connus au niveau de la solidité, c'est à dire sans la fragilité et la complexité des implants multipièces de l'art antérieur.

En d'autres termes l'invention permet de proposer une gamme de tiges d'ancrage intra médullaire d'une grande solidité mais dont la déformation sous contrainte est équivalente à celle de l'os dans le plan sagittal (antéro-postérieur), plan suivant lequel les contraintes physiologiques sont majoritairement transmises lors de la mise en charge de l'articulation (pincement, aggripement,...).

Les phénomènes de contraintes dits de « stress-shielding » (en langue anglo-saxonne) associés aux différences de rigidité entre l'os et les pièces prothétiques sont dès lors minimisés, favorisant l'ostéo-intégration de la tige intramédullaire.

Dans ce but la présente invention propose une tige d'ancrage intra médullaire pour tête d'implant orthopédique, amovible par rapport à la tête avec qui elle comprend des moyens de connexion, ladite tige comprenant un corps au moins en partie évidé longitudinalement, parallèlement ou sensiblement parallèlement au plan dorso-palmaire, caractérisé en ce que la tige est formée d'une seule pièce par ledit corps, et en ce qu'elle présente une forme de pieu tronconique ou sensiblement tronconique en matériau biologiquement compatible.

Grâce à cette modularité, à une forme de la tige allégée dans le plan dorso-palmaire, celle-ci étant fabriquée dans un matériau biologiquement compatible déterminé, la tige est ainsi plus souple dans ce plan et mieux adaptable aux phalanges concernées, ce qui limite l'usure de la prothèse en permettant une accroche meilleure et moins traumatisante de l'os sur la tige.

Particulièrement avantageusement la partie évidée longitudinalement est agencée pour conférer à ladite tige de forme et de matériau déterminés une élasticité et/ou une rigidité correspondantes à des valeurs déterminées proches de celles d'un os, et notamment d'un os de doigt de main ou de pied.

Les valeurs déterminées peuvent être choisies par l'homme du métier en fonction des caractéristiques particulières de l'os du patient à soigner, comme décrit ci-après en référence au module d'Young dit « effectif » ou apparent.

Dans des modes de réalisation avantageux, on a de plus recours à l'une et/ou à l'autre des dispositions suivantes :
- la tige présente un rayon de courbure non nul agencé pour s'adapter à l'intérieur de la cavité médullaire d'une phalange ;
- elle présente une butée d'extrémité anti-enfoncement ;
- la forme de la tige au moins en partie évidée et son matériau lui confèrent un module d'Young effectif E_{eff} < 30 Gpa ;

Par module d'Young effectif ou apparent on entend un module d'Young global de la tige dont le comportement est identique à celui d'un élément osseux.

Dans la pratique, et pour calculer la module d'Young effectif tel qu'envisagé dans la présente invention, on assimile l'os à un tube cortical de module d'Young de l'ordre de 20 Gpa, la partie spongieuse ne participant donc pas ou peu à la résistance.

On transforme ensuite cet os tubulaire en un matériau homogène, c'est à dire en une base pleine qui présente alors ledit module d'Young effectif.

Par exemple pour un os jeune (≤ 35 ans) l'épaisseur du tube est de l'ordre de 1,5 mm, et le module d'Young effectif de la tige pleine équivalente est alors de l'ordre de 16 Gpa.

Pour une personne âgée, l'épaisseur de la corticale est plus proche de 1 mm et le module équivalent est alors de l'ordre de 13 Gpa.

Le module d'Young effectif ou apparent de la tige est donc la combinaison entre le module intrinsèque et un facteur géométrique ;
- le module d'Young effectif est E_{eff} < 20 Gpa, par exemple 16 Gpa ;
- le module d'Young effectif est E_{eff} < 15 Gpa, par exemple 12 ou 13 GPa ;
- la tige présente une section transversale en H ;
- elle présente une section transversale en U ou en V ;
- elle présente une fente en bec de canard dans le plan dorso-palmaire ;
- elle présente une forme évidée en demi-cylindre côté palmaire ou en demi-tube ;
- les moyens de connexion sont formés par un emmanchement conique d'un pion solidaire de la tête dans un évidement ménagé dans la tige correspondante. L'évidement est de forme complémentaire à celle du pion ;
- la tige est dissymétrique par rapport à un plan médian passant par l'axe de l'évidement, parallèle au plan dorso-palmaire ;
- l'emmanchement conique est un cône Morse, c'est à dire avec un angle de conicité de l'ordre de 5 % : mais cette conicité peut également être plus faible, par exemple de l'ordre de 3 % ou 2,5 % ;
- les moyens de connexion entre la tige et la tête correspondante comprennent de plus un évidement ou une rainure formé dans l'une des parties propre à recevoir une plaquette de forme complémentaire solidaire de l'autre partie ;
- la tige comporte au moins en partie un revêtement ostéo-intégrateur augmentant l'ancrage ;
- la tige est en titane micro-poreux et/ou est revêtu d'hydroxyapatite ;
- la tige est ajourée par au moins un évidement traversant transversalement ou sensiblement transversalement ladite tige.

L'invention propose également un implant comprenant une tête d'articulation intra phalangienne comportant une tige telle que décrite ci-dessus.

Avantageusement le matériau de la tige est différent du matériau de la tête d'articulation.

Plus précisément, on peut prévoir deux raideurs apparentes ou modules d'Young effectif de tiges différents selon les patients, soit une tige rigide pour les patients jeunes (i.e. inférieur à de l'ordre de 30 GPa) et une tige plus souple pour les patients âgés (i.e. inférieur à de l'ordre de 15 GPa), la tête étant quant à elle en polyéthylène de module d'Young différent plus faible, par exemple < 5 Gpa, par exemple de 2 Gpa.

Enfin l'invention propose un ensemble d'implants pour lesquels les moyens de connexion sont identiques entre d'une part, plusieurs têtes de tailles différentes et d'autre part, des tiges identiques ou différentes de premier éléments et/ou de second éléments.

Une telle disposition autorise une grande modularité.

L'invention propose aussi un procédé de mise en place d'une tige d'un implant orthopédique pour arthroplastie digitale du type décrit ci-dessus.

Elle sera mieux comprise à la lecture de la description qui suit de modes de réalisation donnés ci-après à titre d'exemples non limitatifs.

La description se réfère aux dessins qui l'accompagnent dans lesquels :
La figure 1 est une vue schématique de dessus, d'un squelette de main dans lequel des tiges d'implant selon l'invention ont été mises en place.
La figure 2 montre en perspective une tige d'implant selon un premier mode de réalisation de l'invention.
Les figures 3 et 4 sont des vues en perspective d'exemples de têtes, bi-convexe et bi-concave, propres à s'adapter à la tige de la figure 2.
La figure 4 est une vue schématique de dessus (ou dorsale) montrant un implant selon l'invention, articulation allongée.
Les figures 5A et 5B sont des schémas en coupe des têtes d'articulation respectivement en position doigt fléchi, et doigt droit, illustrant la flexibilité des tiges propice à la minimisation de l'usure des surfaces d'articulation.
La figure 6 est une vue en coupe latérale d'un élément d'implant comprenant une tige et une tête selon un mode de réalisation de l'invention.
Les figures 7 à 11 montrent en perspective arrière, cinq modes de réalisation de tige pour implant selon l'invention.

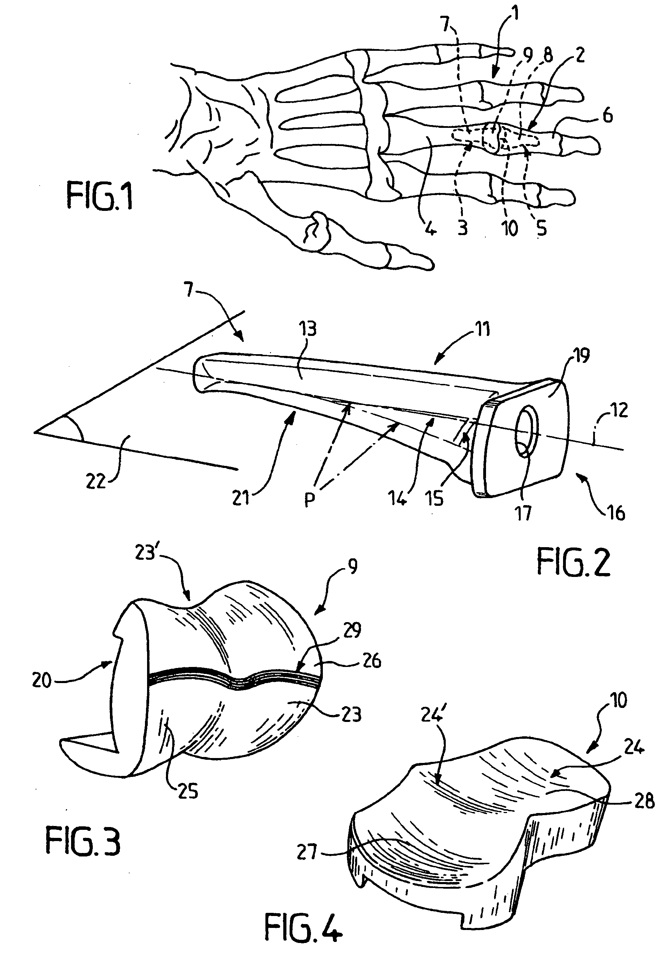

La figure 1 est une vue schématique de dessus d'une main 1 de squelette, comportant des tiges d'implant orthopédique 2 selon l'invention.

L'implant comprend un premier élément 3 pour phalange proximale 4 et un second élément 5 pour phalange distale 6.

Chaque élément comporte respectivement une tige d'implantation ou d'ancrage dans l'os 7, 8 et une tête d'articulation 9, 10 inter-phalangienne.

On utilisera par la suite les mêmes numéros de référence pour désigner des éléments semblables ou identiques.

La figure 2 montre la tige 7 d'ancrage amovible par rapport à la tête 9 du premier élément 3 (cf figure 3) en matériau bio compatible, par exemple en titane.

Elle comporte une partie allongée 11 sensiblement autour d'un axe 12, légèrement courbe, par exemple avec un rayon de courbure p de 100 mm pour une tige pour élément proximal de grande taille et de 90 mm pour élément proximal de moyenne ou petite taille ou encore compris entre 50 mm (grande taille), 40 mm (taille moyenne) et 10 mm (petite taille) pour les éléments distals.

La tige est munie d'une partie 13 agencée pour être introduite dans le trou central de la moelle épinière de la phalange, de forme sensiblement tronconique polygonale, par exemple hexagonale avec une partie élargie d'extrémité 14 à facette 15 permettant une bonne introduction et un blocage latéral en indexation dans la cavité modulaire de la phalange.

La tige 7 est montée de façon amovible par rapport à la tête et comporte des moyens 16 de connexion avec ladite tête.

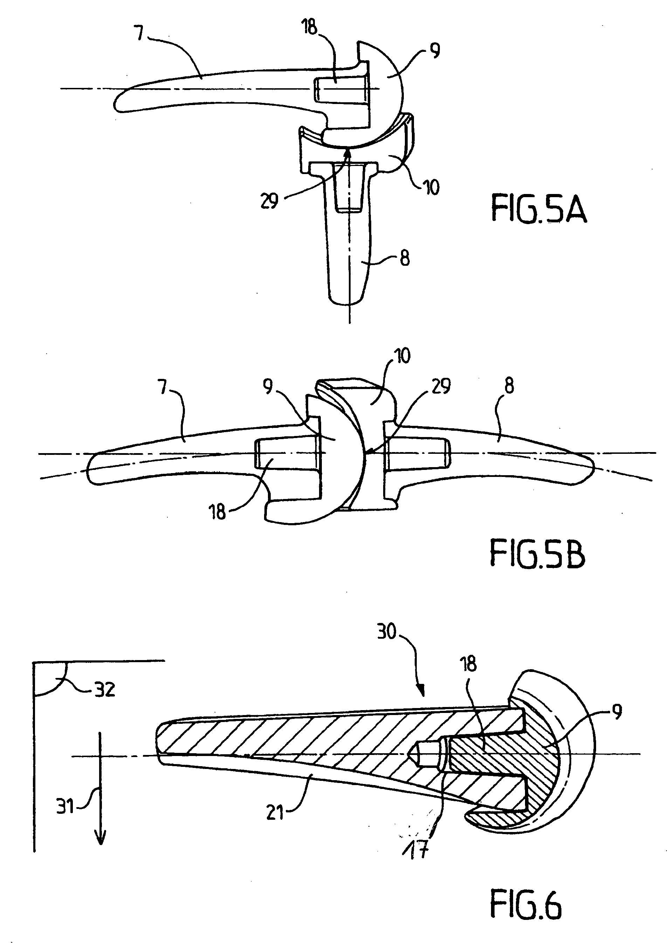

Ces moyens de connexion comportent un orifice 17 conique dans lequel vient s'emmancher un pion conique 18 (cf figure 5A) solidaire de la tête 9, par exemple un cône Morse ou présentant un angle de conicité de 2 à 3 degré.

Pion et évidement sont de formes complémentaires et coopèrent à frottement l'un avec l'autre.

La tige comporte de plus une plaquette 19 anti-enfoncement qui vient coopérer avec un évidement complémentaire en forme de rainure 20 (voir figure 3) ce qui permet lors de l'enfoncement du pion 18 dans l'orifice 17 et blocage de la plaquette 19 dans l'évidement 20, une bonne solidarisation et une bonne indexation de la tête 9 avec la tige 7.

Selon l'invention la tige 7 comporte de plus au moins une partie évidée 21 dans le plan dorso-palmaire 22, qui lui confère la flexibilité recherchée et qui sera détaillée ci-après.

La figure 3 montre la tête 9 du premier élément 3.

Elle présente une surface 23 d'articulation condylienne bi-convexe comportant une vallée centrale 23'.

La figure 4 montre quant-à-elle la tête 10 du second élément.

Celle-ci présente une surface bi-concave 24 agencée pour coopérer avec la surface bi-convexe 21 de la tête du premier élément et munie d'une crête centrale 24'.

Les surfaces 23 et 24 sont par exemple agencées pour être congruentes au niveau des deux condyles 25 et 26 et des surfaces concaves correspondantes 27 et 28 dans le plan frontal de l'articulation et non congruentes dans le plan sagittal.

Mais tous autres types de tête sont parfaitement envisageables.

On a représenté sur la figure 3 la surface de contact obtenue avec une tête de premier élément constituée en matériau élastique lorsqu'une pression longitudinale est exercée.

La ligne de frottement dans le plan frontal se transforme en effet en une surface 29, ce qui permet de mieux répartir les efforts de friction, et de minimiser ainsi l'usure à terme de la tête.

Grâce à la combinaison du matériau de cette tête, qui peut par exemple être un polymère type UHMWPE, des rayons de courbure respectifs mentionnés ci-dessus, et des caractéristiques de l'invention on obtient ainsi un amortissement encore amélioré et une usure minimisée de la prothèse dans le temps.

On constate à ce sujet que la zone de contact 29 balaye la moitié du condyle mais reste à peu près au même endroit sur la partie distale.

Avantageusement la surface la plus fragile est alors choisie coté condyle, la flexibilité des tiges dans le plan dorso-palmaire autorisant par ailleurs une usure encore mieux répartie comme indiqué ci-avant.

On a représenté sur les figures 5A et 5B l'évolution de la flexibilité des tiges 7 et 8 au cours du mouvement, doigt fléchi (figure 5A) et doigt droit (figure 5B).

Le fait d'uniformiser et de standardiser les dimensions des pions 18 et des orifices 17 permet par ailleurs d'adapter indifféremment une tête d'une certaine dimension avec une tige de taille différente ce qui autorise une grande modularité.

D'autres moyens d'assemblage mécanique de la tête sur la tige sont bien-entendu possibles.

La figure 6 est une vue en coupe d'un premier élément 30 comprenant la tête 9 et la tige 7, le pion 18 s'emmanchant en force dans l'orifice 17.

L'évidement 21 permet une bonne flexibilité (flèche 31) dans le plan 32 perpendiculaire au plan dorso-palmaire 22.

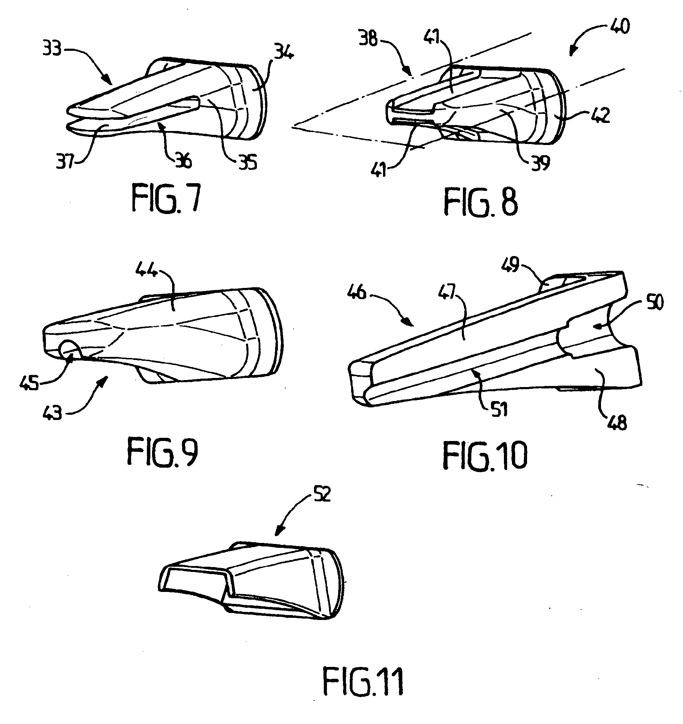

Les figures 7 à 11 montrent des tiges pour élément distal ou proximal de formes et de longueurs différentes selon différents modes de réalisation de l'invention.

La tige 33 de la figure 7 présente d'un côté une plaque 34 anti-enfoncement du type décrit en référence de la figure 2, percée d'un orifice de réception du pion de la tête.

La partie pleine 35 est prolongée dans la direction opposée à la plaque d'une portion 36 en biseau plat munie d'une fente débouchante 37 sur toute sa longueur de faible épaisseur, par exemple 2 mm dans le plan dorsal palmaire, ce qui lui confère une forme normale de bec de canard.

La figure 8 montre un autre mode de réalisation de la tige 38 représentant une partie 39 propre à être enfoncée dans l'os, sensiblement pyramidale ou tronconique de section transversale en forme de H, libérant ainsi de part et d'autre du plan dorso-palmaire 40 des évidements sensiblement parallélépipédiques, de l'extrémité 41 en forme de bec à la plaque 42 anti-enfoncement.

La figure 9 montre une autre tige 43 comprenant une partie 44 de forme tronconique munie sur sa surface inférieure d'une goulotte 45 donnant à la tige une forme évidée sur les 2/3 au 9/10^{ème} de sa longueur.

La figure 10 montre quant à elle en coupe une demi-tige 46 en forme de tube 47 sensiblement cylindrique comprenant une portion d'extrémité 48 munie de la plaque 49, percée d'un évidement 50 pour coopérer avec le pion de la tête comme décrit précédemment, l'évidement 50 étant prolongé d'un alésage 51 sur toute la longueur du tube 47.

La figure 11 montre une tige 52 en forme de tôle pliée, présentant une partie d'extrémité 53 de section transversale en forme de U.

Dans les modes de réalisation de l'invention plus particulièrement décrits ici, les tiges sont rugueuses.

Cette rugosité est par exemple obtenue par sablage ou corindonage, ou par des cannelures transversales (non représentées), permettant d'augmenter l'adhérence dans l'os.

Afin d'améliorer encore l'ancrage un revêtement ostéo-intégrateur est avantageusement prévu par exemple du type Hydroxyapatite (initiales HAP) ou formé en titane micro-poreux par dépôt plasma.

On va maintenant décrire la mise en place d'une prothèse selon l'invention lors d'une opération d'implantation.

Après ouverture du doigt et section des parties osseuses endommagées de façon à mettre en place la prothèse, de façon connue en elle-même, (première étape de résection osseuse) on prépare les logements des tiges (introduction de râpes), puis on introduit dans le canal médullaire la tige de fixation.

On vérifie alors l'espace disponible pour la mise en place des têtes puis on emboîte la tête proximale condylienne 9 qui vient se fixer de façon précise et bloquée par le biais du cône morse sur ladite tige.

On effectue alors un test avec un fantôme de l'autre tête distale.

Puis, après le choix de l'épaisseur en fonction de la résection préalable de l'os, on met en place de l'autre côté, la tige 8 dans la phalange distale et l'articulation est alors finalisée par mise en place de la tête 10.

Le fait que la tête 10 puisse être choisie avec plusieurs épaisseurs différentes pour sa base, permet un ajustage pendant l'opération.

L'ensemble de ces opérations s'effectue de façon connue en elle-même à l'aide d'ancillaires dédiés.

Comme il va de soi et comme il résulte également de ce qui précède, la présente invention n'est pas limitée aux modes de réalisation plus particulièrement décrits. Elle en embrasse au contraire toutes les variantes et notamment celles où les tiges d'ancrage sont non métalliques, par exemple en polyéthylène bio-compatible, ou sont en un autre métal que le titane, celles où la prothèse est une prothèse IP pouce ou gros orteil, ou une prothèse IPD, ou méta-carpophalangienne ce qui implique des dimensions différentes et un dessin des surfaces articulaires différent de façon à autoriser une mobilité latérale adéquate.

## Revendications

1. Implant orthopédique comprenant :
- une tête d'articulation inter-phalangienne (9),
- une tige (2, 7, 8, 33, 38, 43, 46, 52) d'ancrage intra médullaire pour tête d'implant orthopédique, amovible par rapport à la tête (9, 10) avec qui elle comprend des moyens (16) de connexion, ladite tige présentant un corps au moins en partie évidé (21) longitudinalement, parallèlement ou sensiblement parallèlement au plan dorso-palmaire (22),
l'implant étant **caractérisé en ce que** :
- ledit corps présente une forme de pieu tronconique (13) ou sensiblement tronconique,
- la tête (9) comprend un pion conique (18) et un évidement en forme de rainure (20),
- la tige comprend à une extrémité une plaquette (19) anti-enfoncement percée d'un évidement (17, 50), l'évidement (17, 50) s'étendant à l'intérieur de ladite extrémité et étant entouré par la plaquette (19),
l'implant étant en outre **caractérisé en ce que** les moyens (16) de connexion sont formés par :
- un emmanchement conique du pion (18) solidaire de la tête dans l'évidement (17) ménagé dans la tige correspondante, et par
- l'évidement en forme de rainure (20) formé dans la tête qui est propre à recevoir la plaquette (19) qui est de forme complémentaire.

2. Implant selon la revendication 1, **caractérisé en ce que** étant sensiblement allongée autour d'un axe, elle présente un rayon de courbure non nul agence pour s'adapter à l'intérieur de la cavité médullaire d'une phalange.

3. Implant selon l'une quelconque des revendications précédentes, **caractérisée en ce que** la tige présente une butée d'extrémité anti-enfoncement formée par la plaquette (19) anti-enfoncement.

4. Implant selon l'une quelconque des revendications précédentes, **caractérisée en ce que** la tige présente une forme et est constituée en un matériau lui conférant un module d'Young effectif E_{eff} < 30 Gpa.

5. Implant selon l'une quelconque des revendications précédentes, **caractérisée en ce que** la tige présente un module d'Young effectif E_{eff} < 15 Gpa.

6. Implant selon l'une quelconque des revendications précédentes, **caractérisée en ce que** la tige présente une section transversale en H.

7. Implant selon l'une quelconque des revendications 1 à 5, **caractérisée en ce que** la tige présente une section transversale en U ou en V.

8. Implant selon l'une quelconque des revendications 1 à 5, **caractérisée en ce que** la tige présente une fente (37) en bec de canard dans le plan dorso-palmaire.

9. Implant selon l'une quelconque des revendications 1 à 5, **caractérisée en ce que** la tige présente une forme évidée en demi-cylindre cote palmaire ou en demi-tube.

10. Implant selon l'une quelconque des revendications précédentes, **caractérisée en ce que** l'emmanchement conique est un cône Morse.

11. Implant selon l'une quelconque des revendications précédentes, **caractérisé en ce que** la tige est dissymétrique par rapport à un plan médian passant par l'axe de l'évidement, parallèle au plan dorso-palmaire.

12. Implant selon l'une quelconque des revendications précédentes, **caractérisée en ce que** la tige comporte au moins en partie un revêtement ostéo-intégrateur augmentant l'ancrage.

13. Implant selon l'une quelconque des revendications précédentes, **caractérisée en ce que** la tige est en titane micro-poreux et/ou est revêtue d'hydroxyapatite.

14. Implant selon l'une quelconque des revendications précédentes, **caractérisée en ce que** la tige est ajourée par au moins un évidement traversant transversalement ou sensiblement transversalement ladite tige.

15. Implant selon l'une quelconque des revendications précédentes, **caractérisé en ce que** la tête est en un matériau diffèrent de la tige.

16. Implant selon l'une quelconque des revendications précédentes, dans lequel le corps de la tige est dans un matériau biologiquement compatible.

17. Ensemble d'implants selon l'une quelconque des revendications précédentes, **caractérisé en ce que** les implants comportent des moyens de connexion identiques entre d'une part, plusieurs têtes de tailles différentes et d'autre part, des tiges identiques ou différentes.

## Patentansprüche

1. Orthopädisches Implantat, das Folgendes umfasst:
- einen interphalangealen Gelenkkopf (9),
- eine intramedulläre Ankerstange (2, 7, 8, 33, 38, 43, 46, 52) für orthopädische Implantatköpfe, die in Bezug zu dem Kopf (9, 10), mit dem sie Verbindungsmittel (16) umfasst, abnehmbar ist, wobei die Stange parallel oder im Wesentlichen parallel zur dorsovolaren Ebene (22) einen in Längsrichtung zumindest teilweise hohlen Körper (21) aufweist,
wobei das Implantat **dadurch gekennzeichnet ist, dass**:
- der Körper eine Form eines kegelstumpfförmigen oder im Wesentlichen kegelstumpfförmigen Pfahls (13) aufweist,
- der Kopf (9) einen kegelförmigen Stift (18) und eine Aussparung mit der Form einer Rille (20) umfasst,
- die Stange an einem Ende ein Eindringschutzplättchen (19) umfasst, das mit einer Aussparung (17, 50) durchbohrt ist, wobei die Aussparung (17, 50) sich in das Innere des Endes erstreckt und durch das Plättchen (19) umgeben ist, wobei das Implantat ferner **dadurch gekennzeichnet ist, dass** die Verbindungsmittel (16) durch Folgendes gebildet sind:
- eine feste kegelförmige Pressverbindung des Stiftes (18) mit dem Kopf in der Aussparung (17), die in der entsprechenden Stange eingerichtet ist, und durch
- die in dem Kopf gebildete Aussparung in Rillenform (20), die dazu geeignet ist, das Plättchen (19) aufzunehmen, das eine ergänzende Form aufweist.

2. Implantat nach Anspruch 1, **dadurch gekennzeichnet, dass** es, wobei es im Wesentlichen um eine Achse herum länglich ist, einen Krümmungsradius von nicht null aufweist, der eingerichtet ist, um sich an das Innere des medullären Hohlraums eines Fingergliedes anzupassen.

3. Implantat nach einem der vorhergehenden Ansprüche, **dadurch gekennzeichnet, dass** die Stange einen Eidrückschutz-Endanschlag aufweist, der durch das Eindrückschutzplättchen (19) gebildet ist.

4. Implantat nach einem der vorhergehenden Ansprüche, **dadurch gekennzeichnet, dass** die Stange eine Form aufweist und aus einem Material besteht, das ihr ein effektives Elastizitätsmodul E_{eff} < 30 Gpa verleiht.

5. Implantat nach einem der vorhergehenden Ansprüche, **dadurch gekennzeichnet, dass** die Stange ein effektives Elastizitätsmodul E_{eff} < 15 Gpa aufweist.

6. Implantat nach einem der vorhergehenden Ansprüche, **dadurch gekennzeichnet, dass** die Stange einen H-förmigen Querschnitt aufweist.

7. Implantat nach einem der Ansprüche 1 bis 5, **dadurch gekennzeichnet, dass** die Stange einen U- oder V-förmigen Querschnitt aufweist.

8. Implantat nach einem der Ansprüche 1 bis 5, **dadurch gekennzeichnet, dass** die Stange in der dorsovolaren Ebene einen entenschnabelförmigen Schlitz (37) aufweist.

9. Implantat nach einem der Ansprüche 1 bis 5, **dadurch gekennzeichnet, dass** die Stange auf der Palmarseite eine halbzylinderförmig oder halbröhrenformig ausgesparte Form aufweist.

10. Implantat nach einem der vorhergehenden Ansprüche, **dadurch gekennzeichnet, dass** die kegelförmige Pressverbindung ein Morsekegel ist.

11. Implantat nach einem der vorhergehenden Ansprüche, **dadurch gekennzeichnet, dass** die Stange in Bezug zu einer Mittelebene, die durch die Achse der Aussparung parallel zur dorsovolaren Ebene verläuft, asymmetrisch ist.

12. Implantat nach einem der vorhergehenden Ansprüche, **dadurch gekennzeichnet, dass** die Stange zumindest teilweise eine osseointegrierte Beschichtung aufweist, die die Verankerung erhöht.

13. Implantat nach einem der vorhergehenden Ansprüche, **dadurch gekennzeichnet, dass** die Stange aus mikroporösem Titan besteht und/oder mit Hydroxyapatit beschichtet ist.

14. Implantat nach einem der vorhergehenden Ansprüche, **dadurch gekennzeichnet, dass** die Stange durch mindestens eine Aussparung durchbrochen ist, die die Stange in Querrichtung oder im Wesentlichen in Querrichtung durchquert.

15. Implantat nach einem der vorhergehenden Ansprüche, **dadurch gekennzeichnet, dass** der Kopf aus einem unterschiedlichen Material besteht als die Stange.

16. Implantat nach einem der vorhergehenden Ansprüche, wobei der Körper der Stange aus einem biologisch verträglichen Material besteht.

17. Baugruppe von Implantaten nach einem der vorhergehenden Ansprüche, **dadurch gekennzeichnet, dass** die Implantate identische Mittel zur Verbindung zwischen einerseits mehreren Köpfen mit unterschiedlichen Größen und andererseits identischen oder unterschiedlichen Stangen umfassen.

## Claims

1. An orthopedic implant comprising:
- an interphalangeal joint head (9),
- an intramedullary anchor stem (2, 7, 8, 33, 38, 43, 46, 52) for an orthopedic implant head, separable from the head (9, 10) with which it comprises connecting means (16), said stem having a body (21) that is at least partially recessed longitudinally, parallel or substantially parallel to the dorsal-palmar plane (22),
the implant being **characterized in that**:
- said body has a tapered or substantially tapered peg (13) shape,
- the head (9) comprises a conical pin (18) and a recess in the form of a groove (20),
- the stem comprises at one end a sink-preventing small plate (19) pierced with a recess (17, 50), the recess (17, 50) extending inside said end and surrounded by the small plate (19),
the implant being further **characterized in that** the connecting means (16) are formed by:
- a conical interfit between the pin (18) integral with the head in the recess (17) arranged in the corresponding stem, and by
- the recess in the form of a groove (20) formed in the head designed for receiving the small plate (19) of complementary shape.

2. The implant according to claim 1, **characterized in that** being substantially elongated about an axis, it has a non-zero radius of curvature arranged to fit inside the medullary cavity of a phalange.

3. The implant according to any one of the preceding claims, **characterized in that** the stem has a sink-preventing end abutment formed by the sink-preventing small plate (19).

4. The implant according to any one of the preceding claims, **characterized in that** the stem has a shape and is made of a material that provides it with an effective Young's modulus Eeff < 30 Gpa.

5. The implant according to any one of the preceding claims, **characterized in that** the stem has an effective Young's modulus Eeff < 15 Gpa.

6. The implant according to any one of the preceding claims, **characterized in that** the stem has a H-shaped cross-section.

7. The implant according to any one of claims 1 to 5, **characterized in that** the stem has a U- or V-shaped cross-section.

8. The implant according to any one of claims 1 to 5, **characterized in that** the stem has a slot (37) shaped like a duck's bill in the dorsal-palmar plane.

9. The implant according to any one of claims 1 to 5, **characterized in that** the stem has a recessed shape that is semicylindrical on the palmar side or is semitubular.

10. The implant according to any one of the preceding claims, **characterized in that** the conical fitting is a Morse taper.

11. The implant according to any one of the preceding claims, **characterized in that** the stem is asymmetrical with respect to a median plane passing through the axis of the recess, parallel to the dorsal-palmar plane.

12. The implant according to any one of the preceding claims, **characterized in that** the stem includes, at least partially, an osteointegration coating strengthening the anchoring.

13. The implant according to any one of the preceding claims, **characterized in that** the stem is made of microporous titanium and/or is coated with hydroxyapatite.

14. The implant according to any one of the preceding claims, **characterized in that** the stem is cut out with at least one recess transversely or substantially transversely crossing said stem.

15. The implant according to any one of the preceding claims, **characterized in that** the head is made of a different material than the stem.

16. The implant according to any one of the preceding claims, wherein the body of the stem is made of a biologically compatible material.

17. An implant assembly according to any one of the preceding claims, **characterized in that** the implants include identical connecting means between, on the one hand, several heads of different size and, on the other hand, identical or different stems.
